# EUROPÄISCHE PATENTANMELDUNG

(11) **EP 2 589 399 A1**
(43) Veröffentlichungstag der Anmeldung: **08.05.2013**
(21) Anmeldenummer: 11187514.2
(22) Anmeldetag: 02.11.2011
(51) Int. Cl.: A61M 5/315

(54) **Spritze zum Applizieren von Flüssigkeiten**

(71) Anmelder: Uhlmann Pac-Systeme GmbH & Co. KG, 88471 Laupheim (DE)
(72) Erfinder: Bongers-Ambrosius, Hans-Werner, 88471 Laupheim (DE)
(74) Vertreter: Wächter, Jochen

(57) **Zusammenfassung**

Die Spritze zum Applizieren von Flüssigkeiten umfasst einen im Wesentlichen zylindrischen Spritzenkörper (2), einen Kolben (10), der innerhalb des Spritzenkörpers (2) verschiebbar ist, und ein radial umlaufendes Dichtmittel, das sich in einem Zwischenraum zwischen einer Innenseite (14) des Spritzenkörpers (2) und einer Mantelfläche (12) des Kolbens (10) erstreckt. Dabei ist das Dichtmittel als flüssigkeitsundurchlässige schlauchförmige Membran (16) ausgestaltet, die am Spritzenkörper (2) befestigt ist und entweder auch am Kolben (10) befestigt ist oder die Mantelfläche (12) und eine untere Oberfläche (30) des Kolbens (10) umgibt.

## Beschreibung

Die vorliegende Erfindung betrifft eine Spritze zum Applizieren von Flüssigkeiten, wie sie üblicherweise im medizinischen Bereich verwendet wird.

Derartige Spritzen umfassen in der Regel einen im Wesentlichen zylindrischen Spritzenkörper und einen Kolben, der innerhalb des Spritzenkörpers verschiebbar ist. Um eine sichere Abdichtung zwischen der Mantelfläche des Kolbens und der Innenseite des Spritzenkörpers zu gewährleisten, ist üblicherweise eine Dichtung aus Weichkunststoff an der Mantelfläche des Kolbens befestigt bzw. in diese integriert. Um ein reibarmes Gleiten der Dichtung an der Innenseite des Spritzenkörpers zu gewährleisten, ist die Innenseite des Spritzenkörpers in der Regel silikonisiert, was allerdings zu Beeinflussung des pharmazeutischen Produkts führen kann. Außerdem ist die Dicke der Silikonschicht bei der Herstellung nur schwer reproduzierbar.

Der vorliegenden Erfindung liegt die Aufgabe zugrunde, eine Spritze zum Applizieren von Flüssigkeiten zu schaffen, die einfach und kostengünstig aufgebaut ist und die Nachteile silikonisierter Innenseiten des Spritzenkörpers vermeidet.

Diese Aufgabe wird durch die Merkmale des Anspruchs 1 gelöst.

Erfindungsgemäß weist die Spritze zum Applizieren von Flüssigkeiten einen im Wesentlichen zylindrischen Spritzenkörper auf, außerdem einen Kolben, der innerhalb des Spritzenkörpers verschiebbar ist, und ein radial umlaufendes Dichtmittel, das sich - zumindest in einem nicht eingeschobenen Zustand des Kolbens - in einem Zwischenraum zwischen einer Innenseite des Spritzenkörpers und einer Mantelfläche des Kolbens erstreckt. Dabei ist das Dichtmittel als flüssigkeitsundurchlässige schlauchförmige Membran ausgestaltet, die am Spritzenkörper befestigt ist und entweder auch am Kolben befestigt ist oder die Mantelfläche und eine untere Oberfläche des Kolbens umgibt.

Auf diese Weise kann bei der Spritze auf eine silikonisierte Innenseite des Spritzenkörpers verzichtet werden, und die Spritze weist bei einfachem Aufbau eine hohe Funktionssicherheit auf und ist für die Verabreichung von pharmazeutischen Liquida aller Art geeignet.

Um eine vollständige Entleerung des Spritzeninhalts zu gewährleisten, weist die Membran vorzugsweise eine Länge auf, die mindestens einem maximalen Schubweg des Kolbens innerhalb des Spritzenkörpers entspricht.

Die Bewegung des Kolbens innerhalb des Spritzenkörpers wird dadurch vereinfacht, dass die Membran vorzugsweise aus einem Material geringer Elastizität besteht. Beispiele für das Material der Membran sind gewebeverstärkte Grundmaterialien, welche mit pharmazeutisch zugelassenen Materialien wie z.B. NBR, EPDM etc. beschichtet sind.

Um ein besseres Gleiten zwischen Kolben, Membran und Innenseite des Spritzenkörpers zu gewährleisten, ist die Membran vorzugsweise silikonisiert.

In einer speziellen Ausführungsform kann die Membran mit einer beim Verschieben des Kolbens aktivierbaren Verlängerungsreserve, beispielsweise in Form eines Balgs, ausgestaltet sein, wodurch eine Verlängerung der Membran während des Einschiebens des Kolbens gewährleistet wird.

Im Sinne einer vollständigen Abdichtung ist die Membran vorzugsweise an einer ersten radial umlaufenden Befestigungslinie am Kolben befestigt.

Beispielsweise ist diese radial umlaufende erste Befestigungslinie im Bereich der Mantelfläche des Kolbens angeordnet. Alternativ kann die radial umlaufende erste Befestigungslinie auch im Bereich einer oberen oder unteren Oberfläche des Kolbens angeordnet sein.

Zur sicheren Aufnahme der Membran weist der Kolben vorzugsweise eine radial umlaufende Ausnehmung zur Aufnahme eines ersten Endes der Membran und zur Bildung der ersten Befestigungslinie auf.

Ebenso ist die Membran vorzugsweise an einer zweiten radial umlaufenden Befestigungslinie am Spritzenkörper im Bereich dessen oberen Endes befestigt.

Hierbei ist es vorteilhaft, wenn der Spritzenkörper einen radial umlaufenden, nach außen abragenden Vorsprung zur Befestigung eines zweiten Endes der Membran und zur Bildung der zweiten Befestigungslinie aufweist.

Es kann für verschiedene Anwendungsformen auch vorteilhaft sein, wenn die Membran eine nach unten geschlossene Schlaufe mit einem inneren Schlaufenabschnitt und einem äußeren Schlaufenabschnitt bildet derart, dass beim Verschieben des Kolbens nach unten der innere Schlaufenabschnitt auf dem äußeren Schlaufenabschnitt entlang gleitet. Auf diese Weise findet beim Verschieben des Kolbens ein Gleiten ausschließlich auf den beiden Schlaufenabschnitten statt, ohne dass die Innenseite des Spritzenkörpers oder die Mantelfläche des Kolbens Reibung ausgesetzt sind.

Weitere Merkmale und Vorteile der vorliegenden Erfindung ergeben sich aus der nachfolgenden Beschreibung unter Bezugnahme auf die Zeichnungen.
- Fig. 1: ist eine schematische Querschnittsansicht einer ersten Ausführungsform der erfindungsgemäßen Spritze;
- Fig. 2: ist eine schematische Querschnittsansicht einer zweiten Ausführungsform der erfindungsgemäßen Spritze;
- Fig. 3: ist eine schematische Querschnittsansicht einer dritten Ausführungsform der erfindungsgemäßen Spritze; und
- Fig. 4: ist eine schematische Querschnittsansicht einer vierten Ausführungsform der erfindungsgemäßen Spritze.

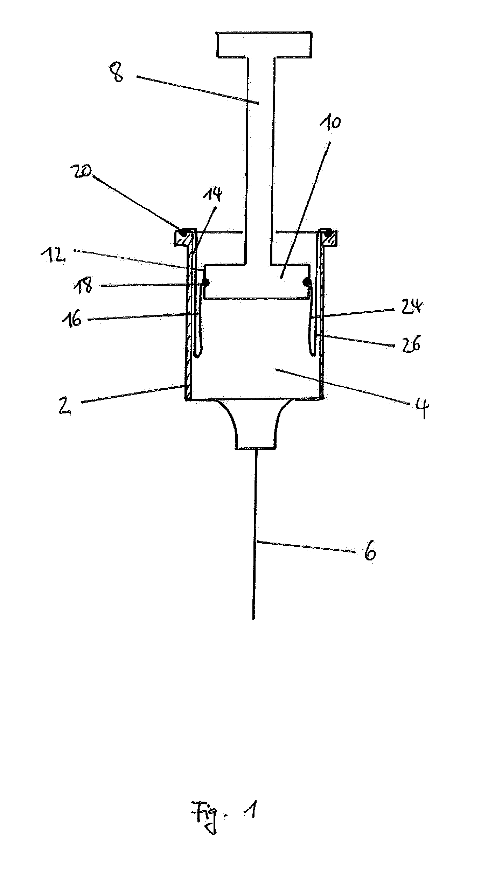

Die in Fig. 1 dargestellte Spritze umfasst einen im Wesentlichen zylindrischen Spritzenkörper 2, der in seinem Inneren einen Hohlraum 4 definiert, der zur Aufnahme der zu applizierenden Flüssigkeit dient. Unter "im Wesentlichen zylindrisch" sollen auch alle Spritzenkörper mit ovalem Querschnitt fallen.

Außerdem umfasst die Spritze eine Nadel 6 sowie ein Schubelement 8 mit einem Kolben 10. Der Kolben 10 ist innerhalb des Spritzenkörpers 2 verschiebbar, um die im Hohlraum 4 aufgenommene Flüssigkeit durch die Nadel 6 herauszudrücken. Der Kolben 10 weist eine Mantelfläche 12 auf, die in einem geringfügigen Abstand von einer Innenseite 14 des Spritzenkörpers 2 entlang derselben verschoben wird.

In sämtlichen Figuren ist der Zwischenraum zwischen der Innenseite 14 des Spritzenkörpers 2 und der Mantelfläche 12 des Kolbens 10 deutlich vergrößert und somit nicht maßstabsgetreu dargestellt. In Wirklichkeit liegt die Mantelfläche 12 des Kolbens 10 eng an der Innenseite 14 des Spritzenkörpers 2 an und ist von dieser lediglich durch ein dazwischen liegendes, radial umlaufendes Dichtmittel beabstandet.

Dieses radial umlaufende Dichtmittel, das sich in dem Zwischenraum zwischen der Innenseite 14 des Spritzenkörpers 2 und der Mantelfläche 12 des Kolbens 10 erstreckt, ist als flüssigkeitsundurchlässige schlauchförmige Membran 16 ausgestaltet, die sowohl am Kolben 10 als auch am Spritzenkörper 2 befestigt ist.

Die Membran 16 weist dabei vorzugsweise eine Länge auf, die mindestens einem maximalen Schubweg des Kolbens 10 innerhalb des Spritzenkörpers 2 entspricht. Die Membran 16 besteht aus einem Material geringer Elastizität, beispielsweise aus einem Gewebe, das mit NBR, EPDM oder einem anderen pharmazeutisch zugelassenen Material beschichtet ist. Die Membran besitzt eine Dicke von vorzugsweise zwischen 0,1 bis 0,3 mm. Sie kann auch auf einer oder auf beiden Seitenflächen silikonisiert sein, wenn dies für die Gleitfunktion nützlich ist.

In dem in Fig. 1 dargestellten Beispielsfall ist die Membran 16 an einer ersten radial umlaufenden Befestigungslinie 18 am Kolben 10 befestigt. Diese radial umlaufende erste Befestigungslinie 18 ist hierbei im Bereich der Mantelfläche 12 des Kolbens 10 angeordnet. Hierzu ist das Ende der Membran 16 vorzugsweise in einer radial umlaufenden Ausnehmung in der Mantelfläche 12 des Kolbens 10 versenkt und dort entweder durch Eigenspannung der Membran 16 oder mittels geeigneter anderer Befestigungstechniken wie Ultraschallschweißen, Verkleben etc. sicher fixiert.

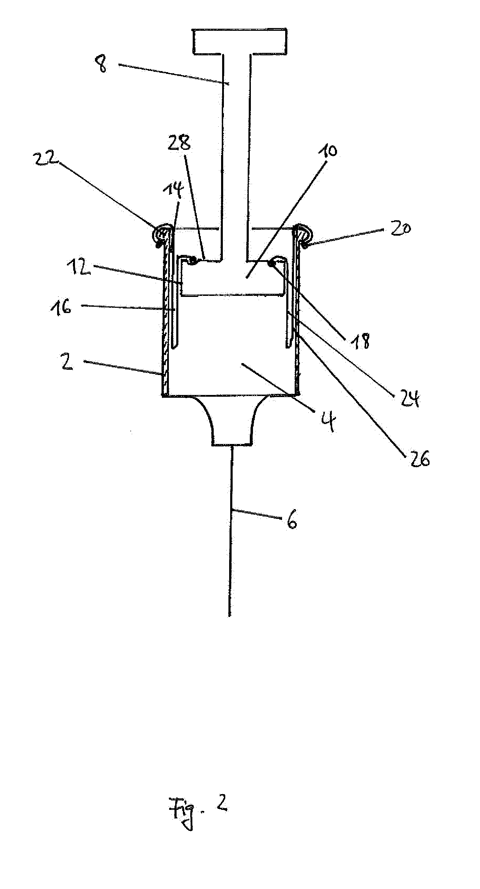

Ebenso ist die Membran 16 an einer zweiten radial umlaufenden Befestigungslinie 20 am Spritzenkörper 2 im Bereich dessen oberen Endes befestigt. In der in Fig. 1 dargestellten Ausführungsform ist hierzu wiederum am oberen Endabschnitt des Spritzenkörpers 2 eine radial umlaufende Ausnehmung vorgesehen, in welche das zweite Ende der Membran 16 eingefügt und mittels geeigneter Befestigungstechniken fixiert ist. Im Fall der Ausführungsform aus Fig. 1 kommt Ultraschallschweißen oder Verkleben in Frage, während bei der Ausgestaltung der Fig. 2 bis 4 zusätzlich auch eine Befestigung der Membran 16 durch Eigenspannung denkbar ist. In den Ausführungsformen der Fig. 2 bis 4 besitzt der Spritzenkörper 2 nämlich einen radial umlaufenden, nach außen abragenden Vorsprung 22 zur Befestigung des zweiten Endes der Membran 16 und zur Bildung der zweiten Befestigungslinie 20.

Die Membran 16 bildet in der Ausführungsform der Fig. 1 eine nach unten geschlossene Schlaufe mit einem inneren Schlaufenabschnitt 24 und einem äußeren Schlaufenabschnitt 26 derart, dass beim Verschieben des Kolbens 10 nach unten der innere Schlaufenabschnitt 24 auf dem äußeren Schlaufenabschnitt 26 entlang gleitet.

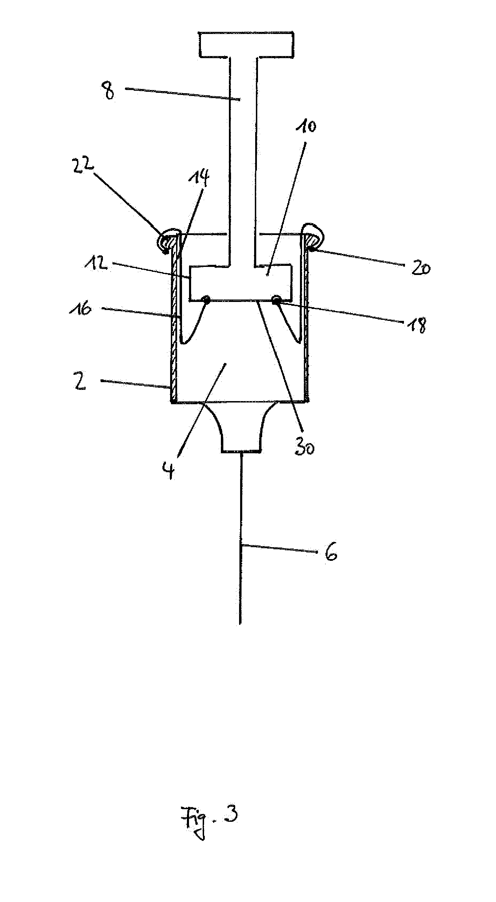

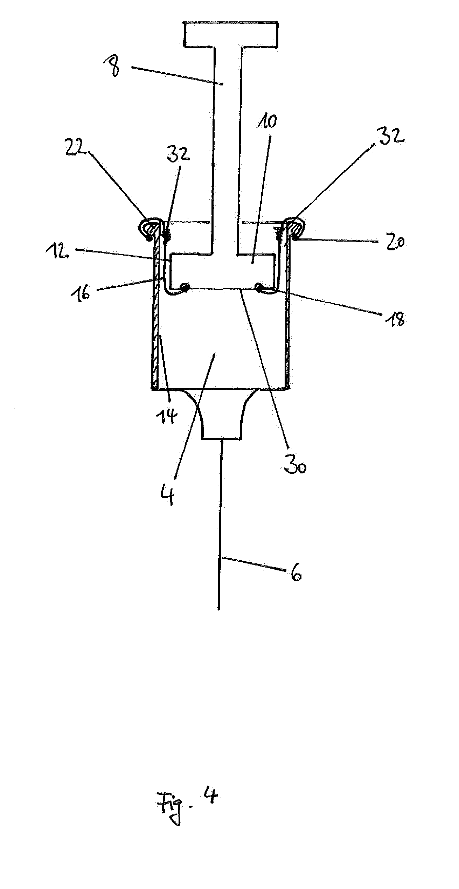

In der in Fig. 2 dargestellten Ausführungsform befindet sich die erste Befestigungslinie 18 der Membran 16 im Bereich einer oberen Oberfläche 28 des Kolbens 10, während sie in den Ausführungsformen der Fig. 3 und 4 im Bereich einer unteren Oberfläche 30 des Kolbens 10 angeordnet ist.

Anders als in Fig. 1 bis 3 ist die Membran 16 in der Ausführungsform der Fig. 4 mit einer beim Verschieben des Kolbens 10 aktivierbaren Verlängerungsreserve 32 versehen, beispielsweise in Form eines Schlaufenbalgs, dessen Windungen leicht miteinander verhaftet sind und sich durch Verschieben des Kolbens 10 voneinander lösen lassen. Ebenso sind andere Verlängerungsreserven denkbar.

In der in Fig. 4 dargestellten Ausführungsform kann die schlauchförmige Membran 16 auch an ihrem unteren Ende geschlossen sein, sodass sie die untere Oberfläche 30 und die Mantelfläche 12 des Kolbens 10 vollständig umgibt. Die Membran 12 ist somit in Form einer Art Rotationsparaboloid ausgebildet. Dies soll ebenfalls unter den Begriff "schlauchförmig" fallen.

Es sind noch viele weitere Modifikationen der erfindungsgemäßen Spritze denkbar. Insbesondere bei Form und Ausgestaltung der Membran 16 sowie deren Befestigung am Spritzenkörper 2 bzw. am Kolben 10 kann unter einer Vielfalt von Möglichkeiten der konkreten Ausgestaltung ausgewählt werden.

## Patentansprüche

1. Spritze zum Applizieren von Flüssigkeiten, mit
einen im Wesentlichen zylindrischen Spritzenkörper (2),
einem Kolben (10), der innerhalb des Spritzenkörpers (2) verschiebbar ist, und
einem radial umlaufenden Dichtmittel, das sich in einem Zwischenraum zwischen einer Innenseite (14) des Spritzenkörpers (2) und einer Mantelfläche (12) des Kolbens (10) erstreckt,
**dadurch gekennzeichnet, dass**
das Dichtmittel als flüssigkeitsundurchlässige schlauchförmige Membran (16) ausgestaltet ist, die am Spritzenkörper (2) befestigt ist und entweder auch am Kolben (10) befestigt ist oder die Mantelfläche (12) und eine untere Oberfläche (30) des Kolbens (10) umgibt.

2. Spritze nach Anspruch 1, **dadurch gekennzeichnet, dass** die Membran (16) eine Länge aufweist, die mindestens einem maximalen Schubweg des Kolbens (10) innerhalb des Spritzenkörpers (2) entspricht.

3. Spritze nach Anspruch 1 oder 2, **dadurch gekennzeichnet, dass** die Membran (16) aus einem Material geringer Elastizität besteht.

4. Spritze nach einem der vorangehenden Ansprüche, **dadurch gekennzeichnet, dass** die Membran (16) aus gewebeverstärktem NBR oder EPDM besteht.

5. Spritze nach einem der vorangehenden Ansprüche, **dadurch gekennzeichnet, dass** die Membran (16) silikonisiert ist.

6. Spritze nach einem der vorangehenden Ansprüche, **dadurch gekennzeichnet, dass** die Membran (16) mit einer beim Verschieben des Kolbens (10) aktivierbaren Verlängerungsreserve (32) ausgestaltet ist.

7. Spritze nach einem der vorangehenden Ansprüche, **dadurch gekennzeichnet, dass** die Membran (16) an einer ersten radial umlaufenden Befestigungslinie (18) am Kolben (10) befestigt ist.

8. Spritze nach Anspruch 7, **dadurch gekennzeichnet, dass** die radial umlaufende erste Befestigungslinie (18) im Bereich der Mantelfläche (12) des Kolbens (10) angeordnet ist.

9. Spritze nach Anspruch 7, **dadurch gekennzeichnet, dass** die radial umlaufende erste Befestigungslinie (18) im Bereich einer oberen Oberfläche (28) des Kolbens (10) angeordnet ist.

10. Spritze nach Anspruch 7, **dadurch gekennzeichnet, dass** die radial umlaufende erste Befestigungslinie (18) im Bereich der unteren Oberfläche (30) des Kolbens (10) angeordnet ist.

11. Spritze nach einem der vorangehenden Ansprüche, **dadurch gekennzeichnet, dass** der Kolben (16) eine radial umlaufende Ausnehmung zur Aufnahme eines ersten Endes der Membran (16) und zur Bildung der ersten Befestigungslinie (18) aufweist.

12. Spritze nach einem der vorangehenden Ansprüche, **dadurch gekennzeichnet, dass** die Membran (16) an einer zweiten radial umlaufenden Befestigungslinie (20) am Spritzenkörper (2) im Bereich dessen oberen Endes befestigt ist.

13. Spritze nach Anspruch 12, **dadurch gekennzeichnet, dass** der Spritzenkörper (2) einen radial umlaufenden, nach außen abragenden Vorsprung (22) zur Befestigung eines zweiten Endes der Membran (16) und zur Bildung der zweiten Befestigungslinie (20) aufweist.

14. Spritze nach einem der vorangehenden Ansprüche, **dadurch gekennzeichnet, dass** die Membran (16) eine nach unten geschlossene Schlaufe mit einem inneren Schlaufenabschnitt (24) und einem äußeren Schlaufenabschnitt (26) bildet derart, dass beim Verschieben des Kolbens (10) nach unten der innere Schlaufenabschnitt (24) auf dem äußeren Schlaufenabschnitt (26) entlanggleitet.
